# EUROPEAN PATENT APPLICATION

(11) **EP 4 364 672 A1**
(43) Date of publication of application: **08.05.2024**
(21) Application number: 22849600.6
(22) Date of filing: 28.07.2022
(51) Int. Cl.: A61B 8/12, A61B 1/00

(54) **CATHETER FOR DIAGNOSTIC IMAGING**

(30) Priority: 30.07.2021 JP 2021126235
(71) Applicant: TERUMO Kabushiki Kaisha, Tokyo 151-0072 (JP)
(72) Inventor: KAKIMOTO Yasuhiro, Ashigarakami-gun, Kanagawa 259-0151 (JP); FUKUDA Tomohiro, Ashigarakami-gun, Kanagawa 259-0151 (JP)
(74) Representative: Casalonga
(86) International application number: PCT/JP2022/029205
(87) International publication number: WO 2023/008539

(57) **Abstract**

An image diagnosis catheter includes: an outer tube; a first support tube disposed on the radially inner side of the outer tube; a drive shaft disposed on the radially inner side of the first support tube; an inner tube disposed on the radially inner side of the outer tube and radially outer side of the first support tube; and a second support tube disposed on the radially inner side of the inner tube and radially outer side of the drive shaft, being axially movable with the drive shaft and the inner tube and relative to the outer tube and the first support tube.

## Description

### Technical Field

This disclosure relates to an image diagnosis catheter.

### Background Art

An image diagnosis catheter typically has a pull-back mechanism on a side closer to a user's hand for changing a relative position between a sheath and a drive shaft in order to continuously monitor a cross section of a body cavity. The pull-back mechanism includes an outer tube and an inner tube that is disposed on the radially inner side of the outer tube and radially outer side of the drive shaft and is axially movable with the drive shaft and relative to the outer tube.

During push-forward operation with respect to the pull-back mechanism in which the inner tube is pushed into the outer tube, when the drive shaft is buckled inside the outer tube, rotation of the drive shaft may cause the drive shaft to be broken and wrenched off. For this reason, for example, JP 4672188 B1 discloses a pull-back mechanism in which a support tube axially movable with an outer tube is disposed on the radially outer side of a drive shaft and radially inner side of an inner tube so as not to buckle the drive shaft during push-forward operation.

### Citation List

### Patent Literature

Patent Literature 1: US Pat. No. 4,672,188

### Summary of Invention

### Technical Problem

However, in a support tube as disclosed in JP 4672188 B1, it is difficult to prevent buckling of a drive shaft inside an inner tube during push-forward operation.

Therefore, an object of this disclosure is to provide an image diagnosis catheter in which a drive shaft is hardly buckled not only inside an outer tube but also inside an inner tube during push-forward operation.

### Solution to Problem

According to an aspect of this disclosure, an image diagnosis catheter includes: an outer tube; a first support tube disposed on the radially inner side of the outer tube; a drive shaft disposed on the radially inner side of the first support tube; an inner tube disposed on the radially inner side of the outer tube and radially outer side of the first support tube; and a second support tube disposed on the radially inner side of the inner tube and radially outer side of the drive shaft, being axially movable with the drive shaft and the inner tube and relative to the outer tube and the first support tube.

As an embodiment of this disclosure, in the image diagnosis catheter, the second support tube overlaps the first support tube in a radial direction at a frontmost position where the inner tube is pushed furthest into the outer tube.

As an embodiment of this disclosure, in the image diagnosis catheter, the second support tube overlaps the first support tube in the radial direction even at a backmost position where the inner tube is drawn furthest from the outer tube.

As an embodiment of this disclosure, in the image diagnosis catheter, the second support tube is disposed on the radially outer side of the first support tube.

As an embodiment of this disclosure, the image diagnosis catheter includes a hub connected to a proximal end of the inner tube, and the second support tube is connected to the hub or the inner tube in an integrated manner.

As an embodiment of this disclosure, in the image diagnosis catheter, the second support tube is disposed on the radially inner side of the first support tube.

As an embodiment of this disclosure, the image diagnosis catheter includes a hub connected to a proximal end of the inner tube, and the second support tube is connected to the hub in an integrated manner.

As an embodiment of this disclosure, in the image diagnosis catheter, the second support tube has a coil shape.

As an embodiment of this disclosure, in the image diagnosis catheter, the first support tube does not overlap the inner tube in a radial direction at a backmost position where the inner tube is drawn furthest from the outer tube.

### Advantageous Effects of Invention

According to this disclosure, it is possible to provide an image diagnosis catheter in which a drive shaft is hardly buckled not only inside an outer tube but also inside an inner tube during push-forward operation.

### BRIEF DESCRIPTION OF DRAWINGS

FIG. 1 is a plan view illustrating an image diagnosis catheter according to a first embodiment to which an external device is connected;
FIG. 2A is a side view illustrating the image diagnosis catheter illustrated in FIG. 1 at a frontmost position before pull-back operation;
FIG. 2B is a side view illustrating the image diagnosis catheter illustrated in FIG. 1 at a backmost position after pull-back operation;
FIG. 3 is a cross-sectional view illustrating a distal end of the image diagnosis catheter illustrated in FIG. 1;
FIG. 4 is a cross-sectional view illustrating a proximal end of the image diagnosis catheter illustrated in FIG. 1;
FIG. 5A is a cross-sectional view illustrating a pull-back mechanism of the image diagnosis catheter illustrated in FIG. 1 at the backmost position;
FIG. 5B is a cross-sectional view illustrating the pull-back mechanism of the image diagnosis catheter illustrated in FIG. 1 at the frontmost position;
FIG. 6 is a cross-sectional view illustrating a modification of the pull-back mechanism at the backmost position;
FIG. 7A is a cross-sectional view illustrating another modification of the pull-back mechanism at the backmost position;
FIG. 7B is a cross-sectional view of the pull-back mechanism illustrated in FIG. 7A at the frontmost position; and
FIG. 8 is a cross-sectional view illustrating another modification of the pull-back mechanism at the backmost position.

### Description of Embodiments

Hereinafter, an embodiment of this disclosure will be described in detail with reference to the drawings.

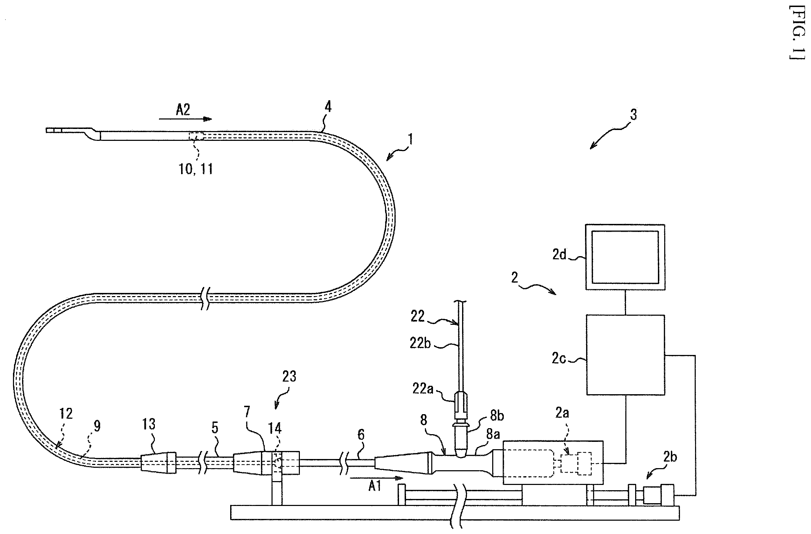

An image diagnosis catheter 1 according to this embodiment illustrated in FIG. 1 is of a dual type employing both intravascular ultrasound (IVUS) and optical coherence tomography (OCT). The dual-type image diagnosis catheter 1 offers three modes, that is, a mode of acquiring a tomographic image by IVUS, a mode of acquiring a tomographic image by OCT, and a mode of acquiring a tomographic image by both IVUS and OCT, and these modes are switched from one to another. As illustrated in FIG. 1, the image diagnosis catheter 1 is connected to an external device 2 and driven. The image diagnosis catheter 1 and the external device 2 constitute an image diagnosis apparatus 3.

The image diagnosis catheter 1 includes a sheath 4 to be inserted into a body cavity such as a vascular channel (for example, coronary arteries or other blood vessels) of a living body, an outer tube 5 connected to a proximal end of the sheath 4, an inner tube 6 inserted into the outer tube 5 and movable forward and backward, a unit connector 7 that is connected to a proximal end of the outer tube 5 and holds the inner tube 6 while allowing the inner tube 6 to move forward and backward, and a hub 8 connected to a proximal end of the inner tube 6. The image diagnosis catheter 1 further includes an imaging core 12 provided with a drive shaft 9, a housing 10 fixed to a distal end of the drive shaft 9, and a signal transmitter and receiver 11 that is housed in the housing 10 and transmits and receives a signal, that is, an ultrasound wave and/or light. The imaging core 12 is inserted into the sheath 4, the outer tube 5, and the inner tube 6 and axially movable forward and backward with the inner tube 6 and relative to the sheath 4 and the outer tube 5.

In this specification, "distal end" signifies an end of the image diagnosis catheter 1 to be inserted into a body cavity, "proximal end" signifies an end of the image diagnosis catheter 1 to be held outside the body cavity, "axially (or axial direction)" signifies a direction along a central axis O of the drive shaft 9 (that is, a direction in which the drive shaft 9 extends), "radially (or radial direction)" signifies a direction along a straight line perpendicular to the central axis O, and "circumferential direction" signifies a direction around the central axis O.

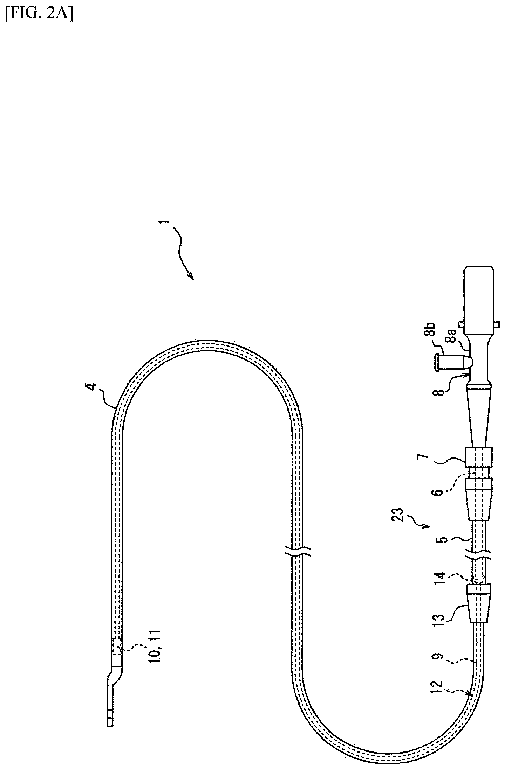

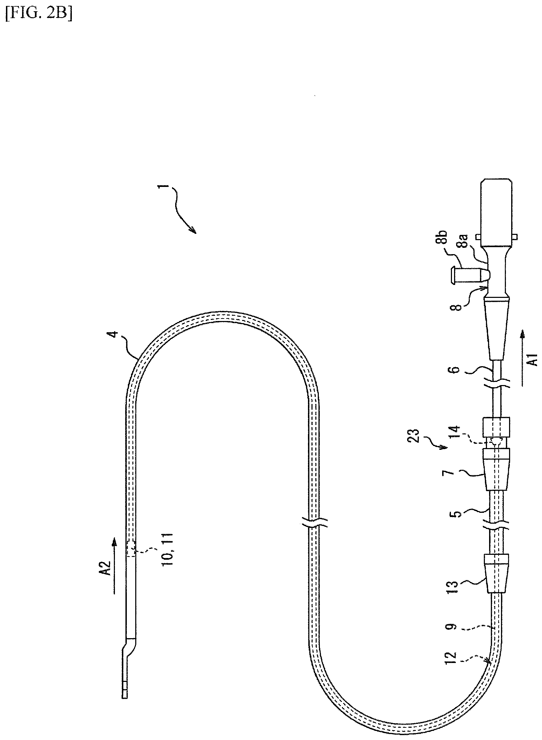

As illustrated in FIG. 2A, the drive shaft 9 extends to the inside of the hub 8 through the sheath 4, the outer tube 5, and the inner tube 6. The hub 8, the inner tube 6, the drive shaft 9, the housing 10, and the signal transmitter and receiver 11 are connected to each other and axially movable forward and backward all together relative to the sheath 4 and the outer tube 5. For this reason, for example, when the hub 8 is pushed toward the distal side, that is, when push-forward operation is performed, the inner tube 6 connected to the hub 8 is pushed into the outer tube 5 and the unit connector 7, whereby the drive shaft 9, the housing 10, and the signal transmitter and receiver 11, that is, the imaging core 12 moves forward inside the sheath 4, that is, toward the distal side. For example, when the hub 8 is pulled toward the proximal side, that is, when pull-back operation is performed, the inner tube 6 is drawn out of the outer tube 5 and the unit connector 7 as indicated by arrow A1 in FIGS. 1 and 2B, whereby the imaging core 12 moves toward the proximal side inside the sheath 4 as indicated by arrow A2.

As illustrated in FIG. 2A, when the inner tube 6 is pushed furthest toward the distal side to a frontmost position, a distal end of the inner tube 6 comes close to a relay connector 13. At this time, the signal transmitter and receiver 11 is located at a distal end of the sheath 4 (near a distal-end surface of a lumen of the sheath 4). The relay connector 13 connects the sheath 4 with the outer tube 5.

As illustrated in FIG. 2B, the distal end of the inner tube 6 is provided with a locking portion 14 to prevent disengagement. The locking portion 14 prevents the inner tube 6 from falling off the outer tube 5. The locking portion 14 is configured to be hooked at a predetermined position on an inner wall of the unit connector 7 when the hub 8 is pulled toward the proximal side to a backmost position, that is, when the inner tube 6 is drawn furthest from the outer tube 5 and the unit connector 7.

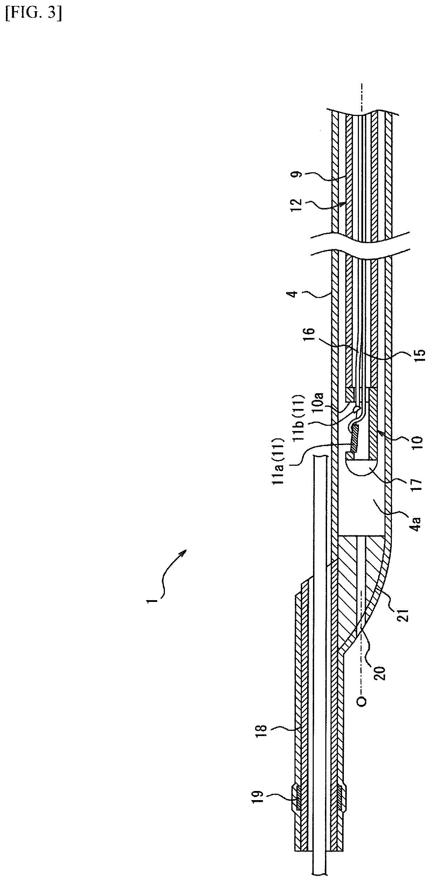

As illustrated in FIG. 3, the drive shaft 9 is an elongated hollow member, and the inside of the drive shaft 9 is provided with an electric signal line (electric cable) 15 and an optical signal line (optical fiber) 16 which are connected to the signal transmitter and receiver 11.

The drive shaft 9 is formed of a coil shaft. Although not illustrated, the coil shaft is formed using, for example, coils with multiple layers having different winding directions. Each coil is made of, for example, metal such as stainless steel or nickel-titanium (Ni-Ti) alloy.

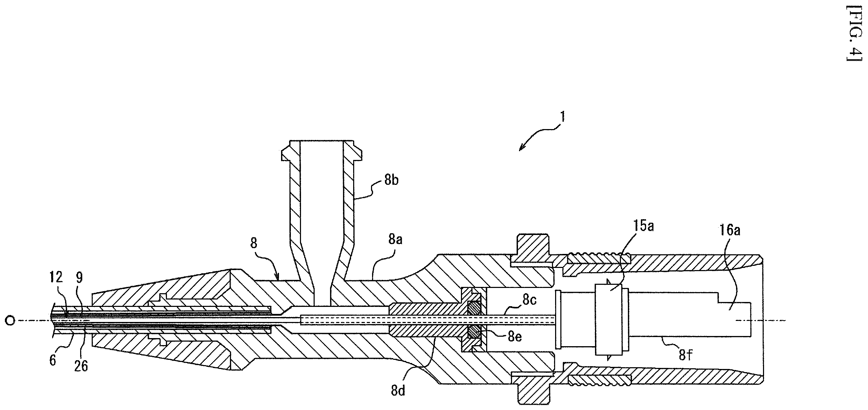

The signal transmitter and receiver 11 includes an ultrasound transmitter and receiver 11a that transmits and receives an ultrasound wave and an optical transmitter and receiver 11b that transmits and receives light. The ultrasound transmitter and receiver 11a includes a transducer that transmits an ultrasound wave into a body cavity based on a pulse signal and receives an ultrasound wave reflected from a biological tissue in the body cavity. The transducer is electrically connected to an electrical connector 15a (see FIG. 4) via the electric signal line 15. The transducer is formed of, for example, a piezoelectric material such as ceramics or quartz.

The optical transmitter and receiver 11b includes an optical element that transmits light into a body cavity and receives light reflected from a biological tissue in the body cavity. The optical element is optically connected to an optical connector 16a (see FIG. 4) via the optical signal line 16. The optical element is formed using, for example, a lens such as ball lens.

The signal transmitter and receiver 11 is housed in the housing 10. A proximal end of the housing 10 is fixed to the distal end of the drive shaft 9. The housing 10 is formed of a cylindrical metallic tube and has a peripheral surface provided with an opening 10a so as not to hinder the progress of a signal transmitted and received by the signal transmitter and receiver 11. The housing 10 is formed by, for example, laser processing. Note that the housing 10 may be formed by shaving a metal lump, metallic powder injection molding (MIM), or the like.

A distal end of the housing 10 is provided with a distal end member 17. The distal end member 17 has a substantially hemispherical outer shape so as not to cause friction against or not to hit an inner surface of the sheath 4. Note that the distal end member 17 may be omitted.

The sheath 4 has the lumen 4a into which the drive shaft 9 is inserted while being movable forward and backward. To the distal end of the sheath 4, a tubular guide wire insertion member 18 that allows passage of a guide wire is attached while being shifted from an axial center of the lumen of the sheath 4. The sheath 4 and the guide wire insertion member 18 are bonded by welding or the like. The guide wire insertion member 18 is provided with a marker 19 having X-ray contrast properties. The marker 19 includes a pipe made of metal having high X-ray impermeability such as Pt or Au.

The distal end of the sheath 4 is provided with a communication hole 20 that allows communication between the inside and outside of the lumen 4a. Furthermore, a reinforcing member 21 joined to the guide wire insertion member 18 is disposed at a distal end of the lumen 4a of the sheath 4. The reinforcing member 21 has a through-hole that allows communication between the communication hole 20 and the lumen 4a disposed closer to the proximal side than the reinforcing member 21. Note that the distal end of the sheath 4 is not necessarily provided with the reinforcing member 21.

The communication hole 20 is a priming solution discharge hole for discharging a priming solution. When the image diagnosis catheter 1 is used, at the time of priming for filling the sheath 4 with the priming solution, the priming solution is released from the communication hole 20 to the outside so as to discharge a gas such as air from the inside of the sheath 4 together with the priming solution.

A distal end portion of the sheath 4, or an area in which the signal transmitter and receiver 11 moves in the axial direction of the sheath 4, is formed into a window having higher permeability than other portions. The sheath 4, the guide wire insertion member 18, and the reinforcing member 21 are formed of flexible materials. Examples of the materials include, but are not particularly limited to, various thermoplastic elastomers based on styrene, polyolefin, polyurethane, polyester, polyamide, polyimide, polybutadiene, trans-polyisoprene, fluoro-rubber, or chlorinated polyethylene. These examples may be used independently, or two or more of these examples (such as polymer alloy, polymer blend, and laminate) may be combined.

As illustrated in FIG. 4, the hub 8 includes a hub body 8a having a tubular shape coaxial with the inner tube 6 and detachably attached to the external device 2 in an integrated manner, a port 8b protruding from the hub body 8a toward the radially outer side and communicated with the inside of the hub body 8a, a connecting pipe 8c attached to an outer peripheral surface of the drive shaft 9 in an integrated manner, a bearing 8d rotatably supporting the connecting pipe 8c, a sealing member (first sealing member 8e) that prevents the priming solution from leaking from a gap between the connecting pipe 8c and the bearing 8d toward the proximal side; and a connector 8f provided with the electrical connector 15a and the optical connector 16a and detachably attached to a first drive unit 2a of the external device 2 in an integrated manner. The connector 8f rotates the connecting pipe 8c and the drive shaft 9 in an integrated manner.

The proximal end of the inner tube 6 is connected to a distal end of the hub body 8a in an integrated manner. The drive shaft 9 is drawn out of the inner tube 6 inside the hub body 8a.

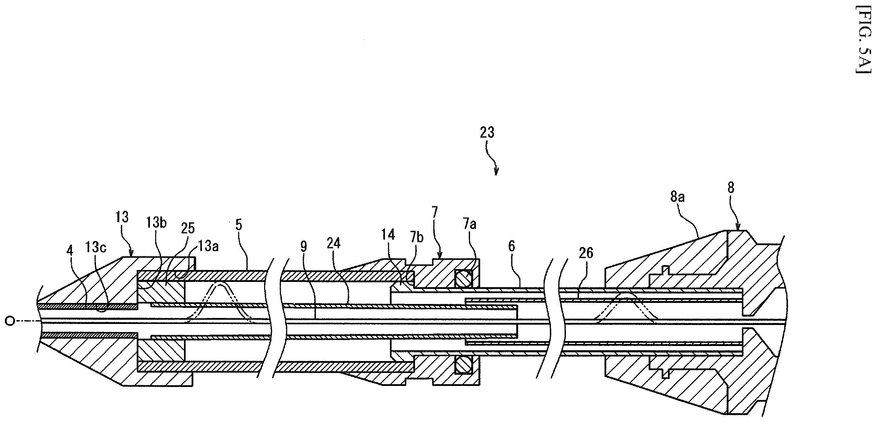

As illustrated in FIG. 1, an injection device 22 that injects the priming solution during the priming is connected to the port 8b. The injection device 22 includes a connector 22a connected to the port 8b and a syringe (not illustrated) connected to the connector 22a via a tube 22b. The priming is typically performed at the backmost position (see FIGS. 1 and 5A).

The external device 2 includes the first drive unit 2a for driving the drive shaft 9 to rotate and a second drive unit 2b for axially moving the drive shaft 9 (that is, for push-forward operation and pull-back operation). The first drive unit 2a includes, for example, an electrical motor. The second drive unit 2b includes, for example, an electrical motor and a direct motion conversion mechanism. The direct motion conversion mechanism converts rotational motions into linear motions and includes, for example, a ball screw and a rack-and-pinion mechanism.

Operations of the first drive unit 2a and the second drive unit 2b are controlled by a control device 2c electrically connected to the first drive unit 2a and the second drive unit 2b. The control device 2c includes a central processing unit (CPU) and a memory. The control device 2c is electrically connected to a display 2d.

A signal received by the ultrasound transmitter and receiver 11a is transmitted to the control device 2c via the electrical connector 15a, subjected to predetermined processing, and shown on the display 2d as an image. A signal received by the optical transmitter and receiver 11b is transmitted to the control device 2c via the optical connector 16a, subjected to predetermined processing, and shown on the display 2d as an image.

At diagnosis, while the sheath 4 is inserted into a body cavity and the imaging core 12 is driven by the first drive unit 2a of the external device 2 to rotate at a constant rotational speed of about 1000 to 10000 rpm, the imaging core 12 moves backward at a constant speed inside the lumen 4a of the sheath 4 due to pull-back operation by the second drive unit 2b of the external device 2. At this time, the control device 2c of the external device 2 causes the signal transmitter and receiver 11 to transmit and receive a signal. A condition of a tissue around the body cavity is shown on the display 2d as an image based on the signal received by scanning by rotating and moving backward.

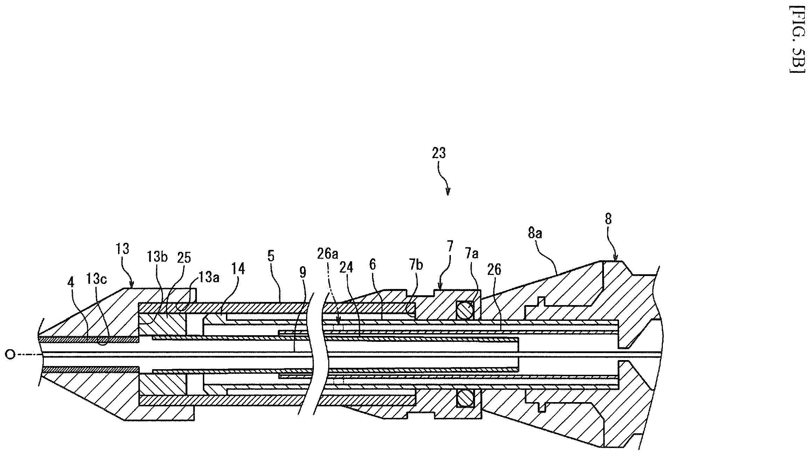

In this manner, the image diagnosis catheter 1 has a pull-back mechanism 23 on a side closer to a user's hand for changing a relative position between the sheath 4 and the drive shaft 9 in order to continuously monitor a cross section of a body cavity. As described in FIGS. 5A and 5B, the pull-back mechanism 23 includes the outer tube 5,' the inner tube 6 disposed on the radially inner side of the outer tube 5 and radially outer side of the drive shaft 9 and axially movable with the drive shaft 9 and relative to the outer tube 5, a first support tube 24 disposed on the radially inner side of the inner tube 6 and radially outer side of the drive shaft 9, a spacer 25 connecting the outer tube 5 and the first support tube 24 in an integrated manner, the relay connector 13, and the unit connector 7. As described above, the relay connector 13 is connected to the sheath 4 in an integrated manner, and the inner tube 6 is connected to the hub 8 in an integrated manner. The unit connector 7 is provided with a sealing member (second sealing member 7a) that prevents the priming solution from leaking from a gap between the unit connector 7 and the inner tube 6 toward the proximal side.

The distal end of the inner tube 6 is provided with the locking portion 14. The locking portion 14 includes an enlarged diameter portion where an outer peripheral surface of the inner tube 6 is enlarged in diameter. A back-end surface of the locking portion 14 protrudes from the outer peripheral surface of the inner tube 6 toward the radially outer side and has an annular shape centering on the central axis O. The unit connector 7 has a stopper surface 7b that abuts on the back-end surface of the locking portion 14 to restrict further backward movement of the inner tube 6. The inner tube 6 has a diameter sufficient to offer sufficient strength to the locking portion 14 as a stopper.

In this embodiment, the pull-back mechanism 23 includes a second support tube 26 disposed on the radially inner side of the inner tube 6 and radially outer side of the drive shaft 9 and axially movable with the drive shaft 9 and the inner tube 6 and relative to the outer tube 5 and the first support tube 24. In this embodiment, the second support tube 26 is disposed on the radially outer side of the first support tube 24. The second support tube 26 is connected to the hub 8 and/or the inner tube 6 in an integrated manner.

The structure for connecting the second support tube 26 and the hub 8 in an integrated manner is not particularly limited. For example, a proximal end of the second support tube 26 may be bonded to the hub 8 directly or via another member with an adhesive or the like. The structure for connecting the second support tube 26 to the inner tube 6 in an integrated manner is not particularly limited. For example, the proximal end or distal end of the second support tube 26 or a portion therebetween may be bonded to the inner tube 6 directly or via another member with an adhesive or the like. For example, a protrusion 26a (see a dashed-two dotted line in FIG. 5B) protruding toward the radially outer side may be disposed in a part of an outer wall of the second support tube 26, and the protrusion 26a and an inner wall of the inner tube 6 may be bonded to each other. The protrusion 26a may be disposed in a part in the circumferential direction or over the entire circumference. According to the structure in which the second support tube 26 and the inner tube 6 are bonded to each other, it is possible to easily ensure the coaxiality of the second support tube 26. With such a structure, when the second support tube 26 moves forward or backward, it is possible to reduce the possibility that the second support tube 26 comes into contact with the first support tube 24 and is hindered from smoothly moving forward and backward. A flanged member may be connected to the proximal end of the second support tube 26 in an integrated manner, and the flanged member may be sandwiched and fixed between the proximal end of the inner tube 6 and the hub 8. An outer peripheral surface of the second support tube 26 may be connected to an inner peripheral surface of the inner tube 6 via one or more axially extending ribs. In this case, the second support tube 26 and the inner tube 6 may be formed in an integrated manner by extrusion molding or the like.

In the pull-back mechanism 23, the outer tube 5, the first support tube 24, the inner tube 6, the drive shaft 9, and the second support tube 26 are coaxial and share the central axis O.

The relay connector 13 has a cylindrical shape, including a proximal-side inner peripheral surface 13a having a columnar shape and a distal-side inner peripheral surface 13c having a columnar shape that is connected to a distal end of the proximal-side inner peripheral surface 13a via an annular stepped portion 13b. An outer peripheral surface of the proximal end of the sheath 4 is joined to the distal-side inner peripheral surface 13c by welding or the like. An outer peripheral surface of a distal end of the outer tube 5 is joined to the proximal-side inner peripheral surface 13a by welding or the like.

The spacer 25 has a tubular shape, an outer peripheral surface of the spacer 25 is in contact with an inner peripheral surface of the distal end of the outer tube 5, and an inner peripheral surface of the spacer 25 is in contact with an outer peripheral surface of a distal end of the first support tube 24. The outer peripheral surface of the spacer 25 is joined to the inner peripheral surface of the distal end of the outer tube 5 by welding or the like, and the inner peripheral surface of the spacer 25 is joined to the outer peripheral surface of the distal end of the first support tube 24 by welding or the like. Furthermore, the spacer 25 is made of, for example, synthetic resin or metal.

The first support tube 24 is formed of, for example, a coil or a tube with a single layer or multiple layers. The first support tube 24 is made of, for example, synthetic resin or metal. Using the coiled first support tube 24 enables the priming solution to pass radially through the first support tube 24, which promotes priming inside the outer tube 5. Instead of the coil shape, the first support tube 24 may be formed into a tubular shape entirely or partially provided with meshed cutouts or the like. The first support tube 24 may be formed into a tubular shape without cutouts. In this case, in order to promote priming the inside the outer tube 5, it is preferable that the spacer 25 is provided with a channel that allows passage of the priming solution.

The second support tube 26 is formed of, for example, a coil or a tube with a single layer or multiple layers. The second support tube 26 is made of, for example, synthetic resin or metal. Using the coiled second support tube 26 enables the priming solution to pass radially through the second support tube 26, which promotes priming inside the inner tube 6. Instead of the coil shape, the second support tube 26 may be formed into a tubular shape entirely or partially provided with meshed cutouts or the like. The second support tube 26 may be formed into a tubular shape without cutouts. In this case, in order to promote priming inside the inner tube 6, it is preferable that the proximal end of the second support tube 26 is connected to the hub 8 or the inner tube 6 via a member forming a channel that allows passage of the priming solution.

When the inner tube 6, the second support tube 26, and the drive shaft 9 move forward relative to the outer tube 5 and the first support tube 24 by push-forward operation (for example, from the backmost position illustrated in FIG. 5A to the frontmost position illustrated in FIG. 5B), the drive shaft 9 is prevented from being buckled inside the outer tube 5 (for example, as indicated by a dashed-two dotted line in FIG. 5A) by the first support tube 24 supporting the drive shaft 9 from the radially outer side, and simultaneously, the drive shaft 9 is prevented from being buckled inside the inner tube 6 (for example, as indicated by a dashed-two dotted line in FIG. 5A) by the second support tube 26 supporting the drive shaft 9 from the radially outer side. Accordingly, it is possible to prevent the drive shaft 9 from being driven to rotate while being buckled inside the pull-back mechanism 23, whereby the drive shaft 9 is prevented from being broken and wrenched off.

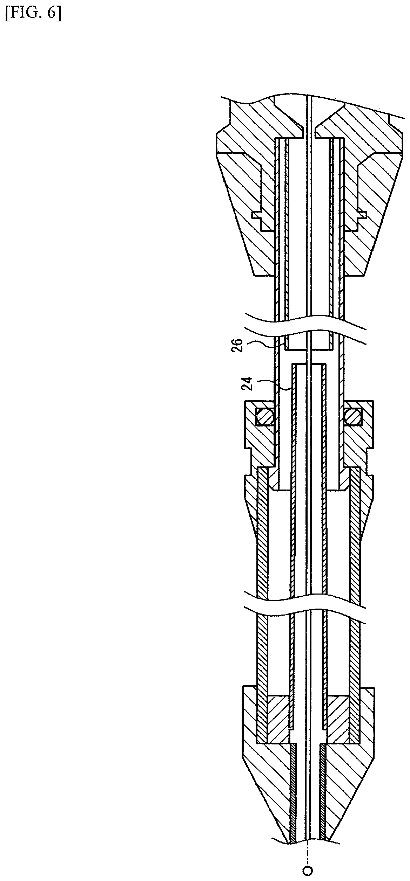

In order to enhance the effect of preventing the buckling of the drive shaft 9, it is preferable that the second support tube 26 overlaps the first support tube 24 in the radial direction at the frontmost position where the inner tube 6 is pushed furthest into the outer tube 5, and it is more preferable that the second support tube 26 overlaps the first support tube 24 in the radial direction even at the backmost position where the inner tube 6 is drawn furthest from the outer tube 5 as in this embodiment. However, the length of the second support tube 26 is set within a range that does not hinder the telescopic action of the pull-back mechanism 23. For example, as in a modification illustrated in FIG. 6, the second support tube 26 does not necessarily overlap the first support tube 24 in the radial direction at the backmost position and may overlap the first support tube 24 at the frontmost position. Alternatively, although not illustrated, the second support tube 26 does not necessarily overlap the first support tube 24 in the radial direction even at the frontmost position.

In this embodiment, since the second support tube 26 is disposed on the radially outer side of the first support tube 24 as described above, the second support tube has an inside diameter larger than that of the first support tube , whereby the buckling of the drive shaft 9 is prevented in a stepwise manner from the proximal side toward the distal side. Preventing the buckling of the drive shaft 9 in a stepwise manner prevents the breakage more efficiently.

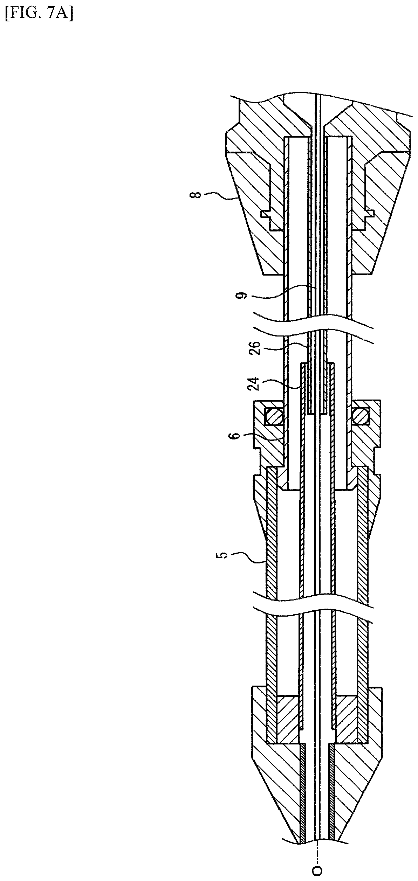

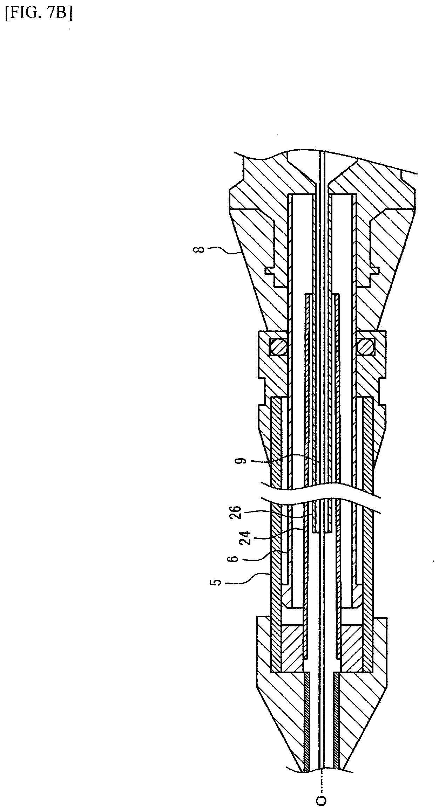

However, as in another modification illustrated in FIGS. 7A and 7B, the second support tube 26 may be disposed on the radially inner side of the first support tube 24. In other words, in this modification, the second support tube 26, the first support tube 24, the inner tube 6, and the outer tube 5 are disposed on the radially outer side of the drive shaft 9 in this order. In this modification, the proximal end of the second support tube 26 is connected to the hub 8 and/or the inner tube 6 in an integrated manner and directly or via another member with an adhesive or the like.

Depending on materials of the first support tube 24 or the second support tube 26, there is a possibility that the drive shaft 9 may be buckled and broken together with the first support tube 24 or the second support tube 26. The buckling is most likely to occur inside the outer tube 5 having a largest space that allows the buckling. In a case where the second support tube 26 is disposed on the radially inner side of the first support tube 24, the first support tube 24 is made to have an increased outside diameter while the second support tube 26 is kept to have a small inside diameter (that is, the first support tube 24 is made to have an increased thickness), thereby enhancing the rigidity inside the outer tube 5. Accordingly, it is possible to prevent the drive shaft 9 from being buckled and broken together with the first support tube 24 inside the outer tube 5.

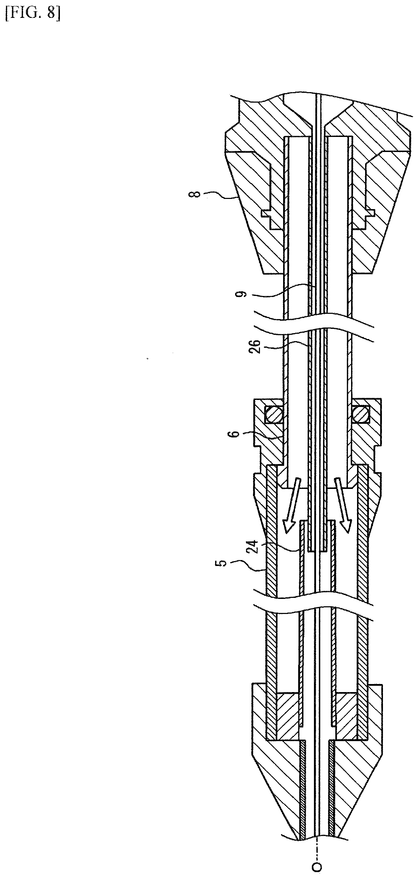

As in another modification illustrated in FIG. 8, the first support tube 24 does not necessarily overlap the inner tube 6 in the radial direction at the backmost position where the inner tube 6 is drawn furthest from the outer tube 5. In other words, in this modification, the proximal end of the first support tube 24 is positioned closer to the distal side than the distal end of the inner tube 6 at the backmost position. According to this configuration, as compared with the configuration illustrated in FIG. 7A in which the first support tube 24 overlaps the inner tube 6 in the radial direction at the backmost position, it is possible to easily ensure a large cross-sectional area at a proximal end of a priming channel formed on the radially outer side of the first support tube 24 when the priming is performed at the backmost position. Accordingly, as indicated by outlined arrows in FIG. 8, the priming solution is easily introduced from the proximal side into a cavity portion surrounded by the inner peripheral surface of the outer tube 5 and the outer peripheral surface of the first support tube 24, or the largest cavity portion on the side closer to a user's hand, and priming is promoted inside the outer tube 5.

In this modification, the second support tube 26 may be on the radially outer side of the first support tube 24. However, as illustrated in FIG. 8, the configuration in which the second support tube 26 is on the radially inner side of the first support tube 24 is preferable because it is easier to obtain the effect of promoting priming.

In this modification, the first support tube 24 does not necessarily overlap the second support tube 26 in the radial direction at the backmost position. However, as illustrated in FIG. 8, it is preferable that the first support tube 24 overlaps the second support tube 26 in the radial direction at the backmost position because it is possible to promote priming inside the first support tube 24.

As described above, according to this embodiment and the modifications thereof, not only the first support tube 24 prevents the buckling of the drive shaft 9 inside the outer tube 5 during push-forward operation but also the second support tube 26 prevents the buckling of the drive shaft 9 inside the inner tube 6 during push-forward operation. Accordingly, it is possible to prevent the drive shaft 9 from being driven to rotate while being buckled inside the pull-back mechanism 23, whereby the drive shaft 9 is prevented from being broken and wrenched off.

This disclosure is not limited to the above embodiments and can be modified in various ways without departing from the gist of the present invention.

Therefore, the image diagnosis catheter 1 according to the embodiment can be changed in various ways as described below.

The image diagnosis catheter 1 according to the embodiment can be changed in various ways as long as the image diagnosis catheter 1 includes: the outer tube 5; the first support tube 24 disposed on the radially inner side of the outer tube 5; the drive shaft 9 disposed on the radially inner side of the first support tube 24; the inner tube 6 disposed on the radially inner side of the outer tube 5 and radially outer side of the first support tube 24; and the second support tube 26 disposed on the radially inner side of the inner tube 6 and radially outer side of the drive shaft 9, being axially movable with the drive shaft 9 and the inner tube 6 and relative to the outer tube 5 and the first support tube 24.

For example, without the spacer 25, the distal end of the first support tube 24 may be directly connected to the relay connector 13 or the outer tube 5 in an integrated manner using an adhesive or the like.

The pull-back mechanism 23 is not limited to the configuration in which the outer tube 5 is connected to the relay connector 13 in an integrated manner and the inner tube 6 is connected to the hub 8 in an integrated manner and may have a configuration in which the outer tube 5 is connected to the hub 8 in an integrated manner and the inner tube 6 is connected to the relay connector 13 in an integrated manner.

The image diagnosis catheter 1 is not limited to a dual-type using both IVUS and OCT and may be of a type using only IVUS or OCT.

In the image diagnosis catheter 1 according to the embodiment, it is preferable that the second support tube 26 overlaps the first support tube 24 in the radial direction at the frontmost position where the inner tube 6 is pushed furthest into the outer tube 5.

In the image diagnosis catheter 1 according to the embodiment, it is preferable that the second support tube 26 overlaps the first support tube 24 in the radial direction even at the backmost position where the inner tube 6 is drawn furthest from the outer tube 5.

In the image diagnosis catheter 1 according to the embodiment, it is preferable that the second support tube 26 is disposed on the radially outer side of the first support tube 24.

In this case, it is preferable that the image diagnosis catheter 1 includes the hub 8 connected to the proximal end of the inner tube 6, and the second support tube 26 is connected to the hub 8 or the inner tube 6 in an integrated manner.

In the image diagnosis catheter 1 according to the embodiment, it is also preferable that the second support tube 26 is disposed on the radially inner side of the first support tube 24.

In this case, it is preferable that the image diagnosis catheter 1 includes the hub 8 connected to the proximal end of the inner tube 6, and the second support tube 26 is connected to the hub 8 in an integrated manner.

In the image diagnosis catheter 1 according to the embodiment, it is preferable that the second support tube 26 has a coil shape.

In the image diagnosis catheter 1 according to the embodiment, it is preferable that the first support tube 24 does not overlap the inner tube 6 in the radial direction at the backmost position where the inner tube 6 is drawn furthest from the outer tube 5.

### Reference Signs List

- 1: Image diagnosis catheter
- 2: External device
- 2a: First drive unit
- 2b: Second drive unit
- 2c: Control device
- 2d: Display
- 3: Image diagnosis apparatus
- 4: Sheath
- 4a: Lumen
- 5: Outer tube
- 6: Inner tube
- 7: Unit connector
- 7a: Second sealing member
- 7b: Stopper surface
- 8: Hub
- 8a: Hub body
- 8b: Port
- 8c: Connecting pipe
- 8d: Bearing
- 8e: First sealing member
- 8f: Connector
- 9: Drive shaft
- 10: Housing
- 10a: Opening
- 11: Signal transmitter and receiver
- 11a: Ultrasound transmitter and receiver
- 11b: Optical transmitter and receiver
- 12: Imaging core
- 13: Relay connector
- 13a: Proximal-side inner peripheral surface
- 13b: Annular stepped portion
- 13c: Distal-side inner peripheral surface
- 14: Locking portion
- 15: Electric signal line
- 15a: Electrical connector
- 16: Optical signal line
- 16a: Optical connector
- 17: Distal end member
- 18: Guide wire insertion member
- 19: Marker
- 20: Communication hole
- 21: Reinforcing member
- 22: Injection device
- 22a: Connector
- 22b: Tube
- 23: Pull-back mechanism
- 24: First support tube
- 25: Spacer
- 26: Second support tube
- 26a: Protrusion
- O: Central axis

## Claims

1. An image diagnosis catheter comprising: an outer tube; a first support tube disposed on the radially inner side of the outer tube; a drive shaft disposed on the radially inner side of the first support tube; an inner tube disposed on the radially inner side of the outer tube and radially outer side of the first support tube; and a second support tube disposed on the radially inner side of the inner tube and radially outer side of the drive shaft, being axially movable with the drive shaft and the inner tube and relative to the outer tube and the first support tube.

2. The image diagnosis catheter according to claim 1, wherein the second support tube overlaps the first support tube in a radial direction at a frontmost position where the inner tube is pushed furthest into the outer tube.

3. The image diagnosis catheter according to claim 2, wherein the second support tube overlaps the first support tube in the radial direction even at a backmost position where the inner tube is drawn furthest from the outer tube.

4. The image diagnosis catheter according to any one of claims 1 to 3, wherein the second support tube is disposed on the radially outer side of the first support tube.

5. The image diagnosis catheter according to claim 4, wherein
the image diagnosis catheter comprises a hub connected to a proximal end of the inner tube, and
the second support tube is connected to the hub or the inner tube in an integrated manner.

6. The image diagnosis catheter according to any one of claims 1 to 3, wherein the second support tube is disposed on the radially inner side of the first support tube.

7. The image diagnosis catheter according to claim 6, wherein
the image diagnosis catheter comprises a hub connected to a proximal end of the inner tube, and
the second support tube is connected to the hub in an integrated manner.

8. The image diagnosis catheter according to any one of claims 1 to 7, wherein the second support tube has a coil shape.

9. The image diagnosis catheter according to any one of claims 1 to 8, wherein the first support tube does not overlap the inner tube in the radial direction at a backmost position where the inner tube is drawn furthest from the outer tube.
